# EUROPEAN PATENT APPLICATION

(11) **EP 3 708 095 A1**
(43) Date of publication of application: **16.09.2020**
(21) Application number: 19161795.0
(22) Date of filing: 11.03.2019
(51) Int. Cl.: A61B 17/42, A61B 17/12

(54) **SURGICAL TOOL**

(71) Applicant: Bornivelli, Christina, 6962 Viganello (CH)
(72) Inventor: Bornivelli, Christina, 6962 Viganello (CH)
(74) Representative: Schaad, Balass, Menzl & Partner AG

(57) **Abstract**

The present invention relates to surgical tool (10) that extends in a longitudinal direction (L) from a proximal end (14) to a distal end (12) and has a length defined by the free distance between said proximal and distal ends 14, 12). It comprises a proximal handle portion (16), which extends along a longitudinal handle axis (LA_{H}) towards a lever portion (20). Said lever portion (20) extends along a longitudinal lever axis (LA_{L}) and is proximally adjacently connected to the handle portion (16) on the one hand and distally adjacently connected to a foot portion (18) on the other hand. The lever portion (20) is thus positioned in between the handle portion (16) and the foot portion (18). The foot portion (18) extends along a longitudinal foot axis (LA_{F}) and includes two distal tongues (22) that extend in the longitudinal direction (L), such that their free distal ends (12) point away from the handle portion (16). The two tongues (22) are positioned at a distance from one another and an underside (30) of the two tongues (22) forms a common concave abutment surface (32) for abutting against a pregnant woman's uterus.

## Description

The present invention relates to a surgical tool for use in a caesarean section procedure.

Caesarean section, also known as C-section or caesarean delivery, is a type of surgery used to deliver a baby. The baby is surgically removed through an incision in the mother's abdomen and then a second incision in the uterus. A caesarean section is often necessary when a vaginal delivery would put the baby or mother at risk. This may include obstructed labour, twin pregnancy, high blood pressure in the mother, breech birth, or problems with the placenta or umbilical cord. A caesarean delivery may be performed based upon the shape of the mother's pelvis or history of a previous C-section.

A C-section typically takes 25 minutes to an hour. It may be done with a spinal block where the woman is awake or under general anesthesia. During the surgical procedure, an incision of about 12 cm (6 inches) is then typically made on the skin of the abdomen. In a next step, the surgeon will separate the abdominal muscles to get to the uterus and a second incision will be made in the uterus itself. After making the uterine incision, the baby is delivered and the placenta is removed from the uterus (optional exteriorization of uterus) before closing the uterine incision, peritoneum and fascia, for example via subcutaneous closure with dissolvable stitches or staples.

Several types of C-section are differentiated by the type of incision (longitudinal or transverse) made on the uterus (hysterotomy), i.e. independent from the incision on the skin: The classical C-section initially was performed with a midline incision through the uterus. While providing a larger space to deliver the baby, it is more prone to complications and associated with a higher rate of maternal morbidity. For that reason, the classical C-section is nowadays only performed at very early gestations, whereas the vast majority of C-sections are performed via a lower uterine segment section that involves either a transverse cut through the uterus just above the edge of the bladder ("Kerr or Monroe-Kerr incision") or a vertical uterine cut ("Kronig or De Lee incision"). Since the latter bears the risk of extension cephalad into the uterine fundus or caudally into the bladder, cervix or vagina, most C-sections these days are performed via a low transverse incision through the uterus. Nevertheless, while having the advantages of reduced blood loss, less need for bladder dissection, easier reapproximation and lower risk of rupture in subsequent pregnancies, lateral extension of a transverse uterine incision is not possible without risking laceration of major blood vessels.

Independent of the chosen hysterotomy technique, the time passing between start of the C-section and delivery of the baby should be kept as short as possible, since many studies have reported a direct association between a prolonged uterine incision-to-delivery time and lower fetal blood gas pH values and Apgar scores - regardless of the type of anaesthesia. It is presently assumed that this is due to hysterotomy induced increased uterine tone, which can interfere with uteroplacental blood flow. Regardless of the reason, it seems that the duration of the skin incision-to-delivery time interval (SIDT) has a significant impact on the well-being of the mother and the baby after C-section. Especially in case of a fetus having a non-reassuring fetal heart rate assessment prior to the onset of surgery, it is crucial that incision-to-delivery time is kept as short as possible.

Any complications occurring during C-sections usually go along with an increase in both time needed to deliver the baby and stress level of the surgeon. Part of the above-mentioned skin incision-to-delivery time (SIDT) is the time passing between the uterine incision and the delivery (UIDT). A shorter UIDT will therefore directly lead to a reduction in SIDT, which, as mentioned above, is believed to have a positive impact on the outcome of the c-section.

The uterus is a muscle that is well supplied with blood and one of the complications that may occur during hysterotomy is excessive bleeding due to the presence of blood vessels at the site of uterine incision (regardless the orientation of the incision itself). Such bleeding may not only prolong UIDT but it may also impair the visibility of the surgical field, which will likely increase the stress level for the surgeon and increase the risk of accidentally injuring the fetus, the bladder, uterine arteries or other structures, thereby increasing both fetal and maternal morbidity. Therefore, controlling the bleeding when making the incision through the uterus is a crucial factor to lower the stress level of the surgeon and decrease the risk of complications.

The problem solved by the present invention is therefore to provide a surgical tool that aids to reduce bleeding during uterine incision in a C-section procedure; thereby allowing for a reduced uterine incision-to-delivery time. This problem is solved by the surgical tool according to independent Claim 1 of the present application. Preferred embodiments are subject of the dependent claims.

The surgical tool of the present invention extends in a longitudinal direction from a proximal end to a distal end and has a length defined by the free distance between said proximal and distal ends. It comprises a proximal handle portion, which extends along a longitudinal handle axis towards a lever portion. Said lever portion extends along a longitudinal lever axis and is proximally adjacently connected to the handle portion on the one hand and distally adjacently connected to a foot portion on the other hand. The lever portion is thus positioned in between the handle portion and the foot portion. The foot portion extends along a longitudinal foot axis and includes a proximal foot area and two tongues extending from said proximal foot area in the longitudinal direction, such that a free end of each tongue is located at the distal end of the tool. The two tongues are positioned at a distance from one another and an underside of the two tongues forms a common abutment surface for abutting against a pregnant woman's uterus.

With the above-described features the inventive tool is especially adapted for providing haemostatic compression to a pregnant woman's uterus in a caesarean section procedure, as will be explained in the following.

The tool of the present invention comes into play after exposing the uterus (i.e. after having made the necessary cuts through the skin and abdominal tissues). Before making the uterine incision, the surgeon or its assistant will place the foot portion of the tool on the pregnant woman's uterus at the location where the incision is to be made. The two tongues will be placed in a longitudinal orientation with respect the uterine incision - in a way that the incision itself is to be made in between the two tongues and with their underside abutting against the uterine tissue. Holding the handle portion in one hand, the surgeon or the assistant will apply pressure against the underlying uterus. While maintaining the pressure, the initial incision (generally 3 to 5 cm in length) will be made in the area in between the two tongues. The size of the tongues and their distance from each other is therefore preferably such that the incision fits inside the area between the two tongues. This way, blood vessels running through the uterine tissue on the site of uterine incision will be compressed in this area and bleeding from blood vessels severed by the cut can be significantly reduced, thereby avoiding obstruction of the surgical field and thus lowering the risk of accidentally injuring the fetus. Apart from improving the surgeons vision, a reduction of bleeding during the uterine incision and consequently overall bleeding will also aid to reducing operating time and stress for the surgeon as well as minimizing epithelial damage and postoperative adhesion, which overall lays the foundation for rapid recovery and fever-free postoperative course after successful C-section.

There are several preferred characteristics of the tool that provide additional benefits:
After the initial cut through the uterus and amniotic sac, the surgeon will usually expand the incision by inserting two fingers (or alternatively by them means of tools, such as forceps) into the opening and spread them apart transversely and vertically to stretch the tissue. This way, the blood vessels and muscles are preferably rather stretched instead of cutting them. An additional benefit of using the fingers to extend the incision is that it reduces the risk of injuring the fetus. To allow for insertion of the surgeon's fingers into the incision while still maintaining the pressure on the uterus by means of the tool, the free distance between the two tongues is preferably such that the index fingers of an average adult person fit in between them. As such, the free distance between the two tongues is preferably at least 1 cm. More preferably, said free distance is at least 1.2 cm and most preferably between 1.5 and 2.5 cm.

The two tongues generally form a U-shape with an area of free space that is open on one side, i.e. at the most distal end of the foot portion. Thanks to this open distal end, the assistant (or the surgeon himself) can simply withdraw the tool - after the surgeon having made the initial cut in the uterine tissue and inserted his finger into the uterus through the initial incision in order to enlarge it sufficiently - without the surgeon having to lift his fingers or tools from the incision site. The surgeon is therefore not impeded by the removal of the tool from the incision site.

To provide a sufficiently large abutment surface - which is formed by the combined undersides of the tongues - each of the two tongues has preferably a width of at least 0.5 cm, preferably at least 0.8 cm. This width also ensures sufficient stability for the tongues and avoids damage to the uterine tissue when the two tongues are pressed against it. Most preferably, the width of each tongue lies within the range of 1 to 3 cm. It is also preferred that this width is essentially constant over the whole length of the tongue. To further reduce the risk of damaging the uterine tissue when being used in accordance with the idea of the present invention, the two tongues have preferably blunt edges and blunt free ends.

As mentioned, the surgeon will set the uterine incision for the caesarean section within the area between the two tongues. Thus, the length of the tongues is preferably such, that the length of the initial uterine incision can be made in between the two tongues. It is therefore preferred that the two tongues each have a length of 4 to 10 cm, preferably about 5 to 8 cm, more preferably about 6 cm.

To most efficiently transfer pressure applied by the surgeon via the tool to a pregnant woman's uterus, the contour of the abutment surface preferably matches the contour of the woman's uterus when said woman the last stages of her pregnancy. In other words, the abutment surface has preferably curvature that matches the curvature of the uterus of a woman that is well advanced in pregnancy. By matching the contour of the abutment surface and the contour of the uterus, a better surface-to-surface contact between the abutment surface and the uterine surface is achieved, which in turn improves the distribution and transmission of force between these two surfaces.

Since the woman's pregnant uterus at time of C-section is generally of a round shape, the abutment surface has preferably the shape of an arc, more preferably a circular arc, when seen in longitudinal section. More specifically, when seen in longitudinal section, the abutment surface preferably includes a circular segment that is enclosed between the arc and its pertaining chord. Said segment has preferably a height of at least 4 mm, more preferably within the range of 5 to 10 mm. The pertaining chord of such circular segment is obviously shorter than the arc and has preferably a length within the range of 4 cm to 10 cm. It is clear that the higher the height of the circular segment, the greater the difference in length between the arc and the chord. For instance, in a specific embodiment the abutment surface has the shape of a circular arc with a cord of 6 cm and a height of 8 mm. Such a curved abutment surface provides an improved contact area between the tool and the uterus and thus enables a particularly effective force transmission and equal force distribution over the entire abutment surface area.

When viewed in longitudinal section, the longitudinal lever axis is preferably disposed at an angle with relation to the longitudinal handle axis. Said angle is preferably at least 10° at the intersection point. More preferably, at the intersection point the two axes enclose an angle α of 15° to 65°. Most preferably, the angle is within the range of 25° to 45°, e.g. 30°. Within the meaning of the present application a "longitudinal section" refers to a cut along a vertical plane that runs along the longitudinal axis. The reason for this preferred angular orientation of the lever portion relative to the handle portion is that it places the handle portion outside the surgeon's working area, meaning that the area above the foot portion is kept free, such that the surgeon has a clear view on the incision site and has enough free space to operate with the scalpel to make the incision in the uterus. While ensuring a free working area for the surgeon, the angular orientation of the handle portion relative to the lever portion shall also allow for an effective transmission of force, i.e. pressure, from handle portion of the surgical tool via the lever and foot portions to the uterus.

For the same reasons, i.e. effective force transmission while providing free access to the surgical site, the lever portion is preferably disposed at an angle relative to the foot portion. More specifically, when viewed in longitudinal section (or when seen in lateral view), the longitudinal lever axis and the longitudinal foot axis are oriented such that they intersect (i.e. they are not parallel or collinear). The angle at the intersection point is preferably at least 60°. More preferably, at the intersection point the lever axis and the foot axis enclose an angle of 75° to 120°. Most preferably, said angle is within the range of 85° to 95°, e.g. 92°. A more or less rectangular orientation of the lever portion relative to the foot portion is therefore particularly preferred.

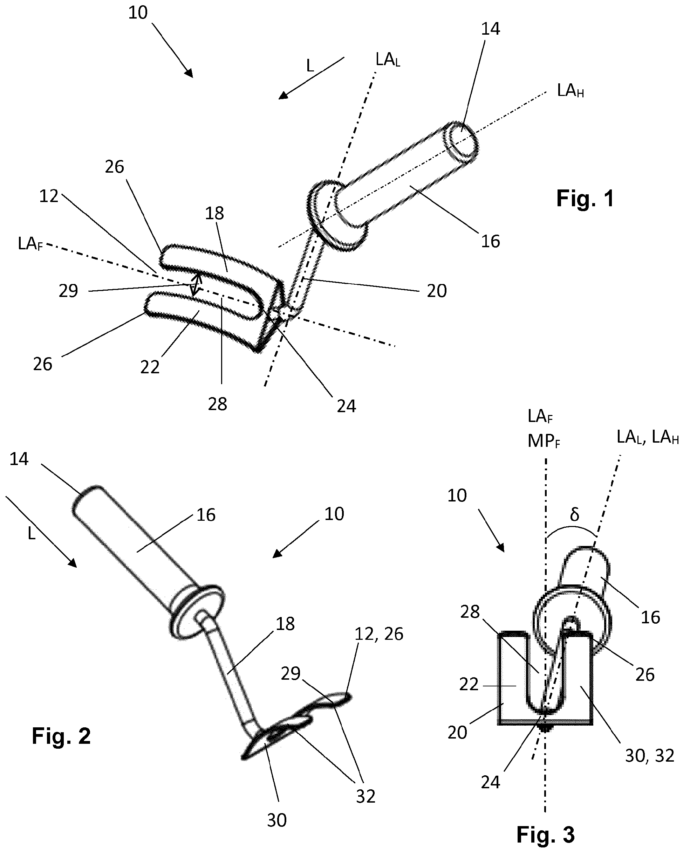

To provide more visual and working space for the surgeon during the incision and to facilitate access to the site of initial uterine incision, the lever portion and the foot portion are preferably connected such that the vertical medial plane along the longitudinal lever axis is offset from the vertical medial plane along the longitudinal foot axis (see Fig. 3), with the two planes intersecting along a vertical line running through the connection point of the foot portion and the lever portion. In other words, in these embodiments, the handle and lever portion on the one hand and the foot portion on the other hand do not share a common vertical longitudinal plane, but the proximal end of the handle portion is positioned at a lateral distance from the longitudinal medial plane MP_{F} through the foot portion. This deviation helps to keep the handle and lever portion out of the surgeon's visual and working field, such that the surgeon's view of the incision site (which is in between the two tongues) is not restricted. When seen in a top view, the lever and handle portions will therefore either point more towards the left side or more towards the right side. Which orientation is preferred depends on the preference of the person holding the tool, e.g. whether this person is left or right handed, and whether the surgeon himself or an assistant standing opposite the operating table is holding the tool during the surgery.

If seen in a lateral view the site of incision is sometimes located more distantly and under the pubic symphysis thus reaching the site of initial uterine incision in this cases can be difficult. This is resolved by displacing the axis of the tongues respect to the axis of the handle.

It is further preferred that the lever portion is rigidly and preferably integrally connected to the handle portion and/or the foot portion. The rigid and preferably integral connection of the portion parts increases the stability of the tool and makes it easier for the surgeon to estimate the amount of pressure that is effectively transferred to the uterus of the pregnant woman. To effectively transfer the pressure applied via the handle portion to the uterine tissue, the tongues are preferably firmly attached or integrally formed with the proximal foot area of the foot portion. To this end, if the surgical tool is made of a metal material, a thickness of the tongues of 1 to 2 mm is generally sufficient, such that they do not break or deform when used within the idea of the present invention. Nevertheless, also when made of metal, the tongues may be thicker, e.g. 1,5 mm, 2 mm or even more.

To provide a good grip for the person holding the tool, the surface of the handle portion may be textured or roughened. Also, the handle portion may be provided with a specific shape that facilitates holding thereof and applying pressure without slipping along the handle. For instance, the handle portion may include a main portion and a distal collar portion, wherein the collar portion is provided with a larger diameter than the main portion to provide a stop surface for the hand of the surgeon (or his assistant) to rest against when transferring pressure via the tool to the pregnant woman's uterus. In a preferred embodiment the handle portion is provided with a main portion having a diameter that continuously increases at the distal end and smoothly transitions into the collar portion. Independent of the presence of a collar portion, the handle portion may have a cross-section that changes along the longitudinal handle axis, e.g. being largest in the middle and continuously decreasing in size towards both ends of the handle portion, such that it fits comfortably into the hand of the user.

In one preferred embodiment, the surgical tool is intended for multiple use and is therefore made of a material that allows facile sterilization, preferably by autoclave. Preferred materials for a multiple-use surgical tool are metals or metal alloys, preferably selected from the group consisting of stainless steel, titanium, tantalum, cobalt and alloys thereof.

In an alternative preferred embodiment, the surgical tool is a disposable tool, i.e. generally intended for one-time use only, such that it can be made from a cost-effective material that provides sufficient stability to the tool and can be sterilized, but not necessarily multiple times. In case of a one-time use tool, it is preferably made of a medical grade plastic material, preferably a medical grade plastic with a limited contact time on tissue ("medical grade" means tested according to USP standards). Plastics that are presently used in the medical field are e.g. PEEK, PA, PTFE, POM, PPSU, PEI, PP, PSU, PC, PPO, TPC, PBT. Since at least some plastic materials are more prone to breaking than metals, the tongues of the surgical tool are in this case preferably at least 0.5 cm thick. More preferably they have each a thickness (height measured in longitudinal section) of at least 0.8 cm, more preferably about 1 cm. It is thereby noted that the tongues do not necessarily need to have a constant thickness, they may also have a thickness that decreases from the point of common origin of the tongues towards their free ends. In this case, the mentioned minimal thickness refers to the part of the tongues that is the least thick.

It is further conceivable to prepare the tongues from a material and with a thickness that allows elastic deformation of the tongues, such that their shape can adapt to the shape of the pregnant woman's uterus.

In another aspect the present invention is also directed to a method of employing a surgical tool as described above in a C-section procedure. Said method comprises the following steps of
a) making an incision in the skin and abdominal tissues of a woman to access the uterus;
b) providing a surgical tool as described in the above sections of the application and placing the foot portion of the tool on the pregnant (woman's) uterus at the location where the initial uterine incision is to be made, such that the underside of the tongues is abutting against the uterine tissue;
c) applying pressure via the handle portion, lever portion and foot portion to the uterus for providing haemostatic compression of the uterine tissue in the area of the later incision;
d) making an initial incision of preferably 3 to 5 cm in length in the uterine tissue within the area between the two tongues;
e) introducing surgeon's fingers or auxiliary tools inside the uterus through the uterine incision;
f) removing the surgical tool from the surgical site
g) extending the uterine incision bilaterally by means of fingers or auxiliary tools;
h) delivering the baby; and
i) closing the uterine and abdominal tissues including the skin.

Before making the uterine incision, any suitable method for entering the peritoneal cavity and provide access to the uterus may be chosen. To give a specific example, the surgeon may use a midline infraumbilical incision, which provides quick access to the uterus. In pregnancy, entry is commonly enhanced by diastasis of the rectus muscles. Although the midline infraumbilical incision is associated with less blood loss, easier examination of the upper abdomen, and easy extension cephalad around the umbilicus, in some cases one might opt for a low transverse incision that can provide easier access and better visualization if there are significant intra-abdominal adhesions from previous operations. Once the uterus is exposed, either a transverse (Monroe-Kerr) or a vertical (Kronig or DeLee) incision may be made in the uterine tissue. The choice of incision is based on several factors, including fetal presentation, gestational age, placental location, and presence of a well-developed lower uterine segment. The incision selected must allow enough room to deliver the fetus without risking injury (either tearing or cutting) to the uterine arteries and veins that are located at the lateral margins of the uterus. In general, a vertical incision is associated with a greater degree of blood loss and a longer operating time than a low transverse incision but poses less risk of injury to the uterine vessels.

Notably, the surgical tool of the present invention can be used for any kind of uterine incision and brings the benefits of reduced blood loss, reduced risk for injuring the baby, shorter operating time and less stress for the surgeon, independent of the chosen incision type. Since longer incision to delivery times are associated with worsening neonatal outcomes, the use of the surgical tool of the present invention in a C-section is likely to also provide better short and long-term outcomes for the baby and the mother.

Preferred embodiments of the present invention will now be described, by way of example only, with reference to the accompanying drawings, in which:
- Fig. 1: shows a top perspective view of an example embodiment of a surgical tool in accordance with the present invention;
- Fig. 2: shows the surgical tool of Fig. 1 in a perspective view;
- Fig. 3: shows the tool of Fig. 1 in a perspective view in the distal-proximal direction;
- Fig. 4: shows the tool of Fig. 1 in a lateral view of; and
- Fig. 5: shows the tool of Fig. 1 in a perspective bottom view.

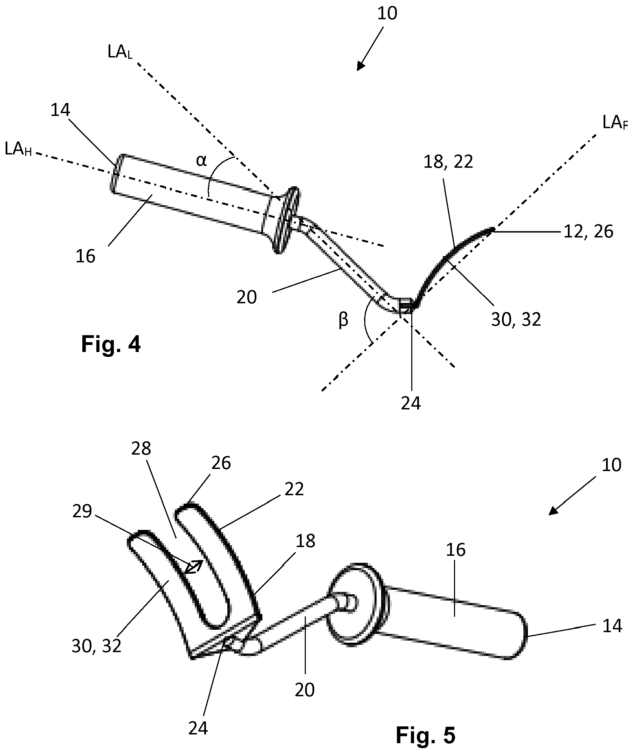

Fig. 1 shows an embodiment of a surgical tool 10 in accordance with the present invention. The tool 10 extends in the longitudinal direction L from a distal end 12 to a proximal end 14 and comprises three main portions; a proximally located handle portion 16, a distally located foot portion 18 and a lever portion 20 that is positioned in between the other two portions 16, 18. Each portion 16, 18, 20 extends along a respective longitudinal axis, i.e. the handle portion 16 along a longitudinal handle axis LA_{H}, the lever portion 20 along a longitudinal lever axis LA_{L} and the foot portion 18 along a longitudinal foot axis LA_{F}. The three axes intersect with each other; thus they do not run in a parallel or co-axial fashion but at an angle in relation to one another. This is best seen in the perspective side-top view of Fig. 1 and the lateral view of Fig. 4.

The tool 10 serves for providing haemostatic compression to a pregnant woman's uterus in a "C-section procedure. More specifically, the size, shape and physical characteristics of the surgical tool 10 are specifically adapted to facilitate the incision making and to reduce blood loss during the C-section. The handle portion 16 of the tool 10 is intended to be held firmly in one hand - of either the surgeon or his assistant. The foot portion 18 comprises two tongues 22 that extend along the longitudinal foot axis LA_{F}, with their common origin being located in a proximal foot area 24 and with their free ends 26 being located at the distal end 12 of the tool 10. With the exception of their common origin, the tongues 22 are spaced apart from one another, such that there is an area of free space 28 in between them. In line with their name they have a tongue 22-like shape, i.e. they are longer than wide but wider than thick.

Each tongue 22 has an underside 30 that is facing away from the handle portion 16. The combined underside area 30 of both tongues 22 together forms a curved abutment surface 32 that is intended to press against the (pregnant woman's) uterus when making the uterine incision: After opening the abdominal cavity and having identified and opened the vesico-uterine fold, the surgeon himself or his assistant will put the surgical tool 10 in place, such that the tongues 22 of the foot portion 18 are placed above and below the level of uterine incision and the underside 30 of the tongues 22 contact the uterine tissue. The area of free space 28 in between the two tongues 22 is sized such that the incision in the uterine tissue can be made within this area. Holding the handle portion 16 in one hand, the surgeon or an assistant will apply a pressure that is directed towards the woman's abdomen with the aim to compress the tissue of the uterus. This way, blood vessels running through uterine tissue in the area of uterine incision will be compressed and bleeding from blood vessels severed when making the incision is significantly reduced.

As is best seen in Figs. 1, 3 and 4, the three portions 16, 18, 20 of the tool 10 are positioned at an angle relative to one another. Specifically, when seen in a frontal view - i.e. when looking onto the underside 30 of the tongues 22 - as shown in Fig. 3, the longitudinal lever axis LA_{L} and the longitudinal handle axis LA_{H} are both running more or less along a common vertical medial plane, yet said common vertical plane is not aligned with the longitudinal medial plane MP_{F} through the foot portion 18 but positioned at an angle δ of about 15° with respect thereto. In other words, the common vertical medial plane through the handle and lever portions 16, 20 deviates from the orientation of the vertical medial plane MP_{F} running along the longitudinal foot axis LA_{F} through the foot portion 18, such that the proximal end 14 of the handle portion 16 is positioned at a lateral distance from said longitudinal middle plane MP_{F}. Said lateral distance is preferably within the range of 10 mm to 17 mm, most preferably about 14 mm. This deviation helps on one hand to keep the handle and lever portion 20 out of the surgeon's visual and working field, such that the surgeon's view of the incision site (which is in between the two tongues 22) is not restricted and on the other hand to manage to reach the uterine incision site more easily, this can be better understood if seen in a lateral view the site of incision is sometimes located more distantly (towards foot of the pregnant woman) and under the pubic symphysis thus reaching the site of initial uterine incision in this cases can be difficult. Whether the lever and handle portions 20, 16 are pointing towards the left or the right side - when seen in a top view - depends on whether the surgeon himself or an assistant is holding the tool 10 and whether this person is left or right handed.

In addition, as shown in Fig. 4, the longitudinal lever axis LA_{L} is positioned at a more or less right angle β with respect to the longitudinal foot axis LA_{F} and at an acute angle α of about 30° with respect to the longitudinal handle axis L_{AH}. This angular orientation of the three portions 16, 18, 20 relative to each other serves two purposes: at the one hand pressure can be effectively transferred from the handle portion 16 towards the foot portion 18. At the other hand, as mentioned above, the lever portion 20 and the handle portion 16 should be kept outside the surgeon's working area when the tool 10 is used to apply pressure on the uterine tissue - by either the surgeon himself or by an assistant, such that the lever and handle portion 16s will neither impair the surgeon when making the incision nor obstructing his visual field of the incision site, but it actually allows the surgeon to better reach the uterine incision site.

As mentioned, the incision through the uterus will be made within the area of free space 28 between the two tongues 22. As such, the length of the tongues 22 is usually about 6 cm. The initial cut is often made shorter than required. Rather than making a longer cut, many surgeons will extend the uterine incision on either side with finger dissection, meaning that the surgeon will insert two fingers into the initial incision and expand it by repetitive stretching or tearing of the tissue. By using the fingers to expand the incision rather than cutting tools it also reduces the risk of inadvertently injuring the fetus. The tool 10 is therefore adapted to allow insertion of the surgeon's finger(s) between the two tongues 22 while still maintaining the pressure on the uterus. (Notably, if the tool 10 is used by the surgeon himself, he will insert one finger after initial incision while pressing the tongues 22 on the uterus. He will then remove the tool 10 and insert a second finger (of his other hand) into the incision in order to extend the uterine incision.) This means that the free distance 29 between the two tongues 22 is preferably such that the index finger or thumb of an average adult person fits therebetween. In terms of specific lengths, the free distance d between the two tongues 22 is generally at least 1 cm, more specifically about 2 cm (Fig. 5).

In addition, the open-end design of the foot portion 18, meaning that the two tongues 22 form together the shape of an U, allows withdrawal of the tool 10 from the surgical site by pulling it away laterally, i.e. horizontally in a direction away from the distal free ends 26 of the tongues 22, while the surgeon can keep his finger in the opening of the uterine tissue.

The foot portion 18 is not flat but curved, such that at least a main portion of the foot portion 18 has a concave shape, in particular the shape of a circular arc when seen in longitudinal section (Fig. 4). The curvature of the arc is adapted to match the average curvature of the uterus of a pregnant woman in her last stages of pregnancy. As such, the whole underside 30 of the tongues 22, which forms the abutment surface 32, will ideally be in full contact with the uterine tissue, thus allowing efficient transfer of force.

The handle portion 16 of the tool 10 is adapted for being held securely by one hand. The length of the handle portion 16 is therefore preferably about 9-12 cm and it has an essentially circular cross-sectional contour with a diameter of preferably about 2-3 cm.

The lever portion 20 is generally smaller in diameter than the handle portion 16. In the shown embodiment, it has a circular cross-section and a diameter of about 1 cm, however, it may also have a rectangular, polygonal or any other cross-sectional shape. The length of the lever portion 20 is within the range of the length of the handle portion 16, or a bit shorter, e.g. about 8-12 cm.

In the depicted embodiment, the tongues 22 have a length within the range of 6-8 cm, a width of about 1.5 cm and a thickness of 1-2 mm. As mentioned, the length of the tongues 22 should allow making the initial cut in the uterine tissue within the area of free space 28 between the two tongues 22. The axial free distance 29 between the two tongues 22 is usually in the range of 1.5-2.5 cm.

As regards the thickness of each portion and in particular of the tongues 22, this variable is inter alia dependent from the chosen material. For instance, if the surgical tool 10 is made of a metal material, 1 or 2 mm thickness will generally be sufficient for the tongues 22 for avoiding breakage or deformation thereof when using the tool 10 within the idea of the described invention. On the other hand, if the tool 10 is made of a plastic material, it may be necessary to increase the thickness of the tongues 22, e.g. up to 1 cm, to insure sufficient stability.

Independent of the material used, the thickness of the tongues 22 does not need to be constant over their entire length. For instance, they may have a thickness that decreases towards their free ends 26. This is particularly preferred in case of the tongues 22 being made from a plastic material.

The portions of the surgical tool 10 may be manufactured from a single piece of plastic or metal, or consist of multiple pieces of plastic or metal that are coupled (e.g. welded or adhered) together.

## Claims

1. Surgical tool (10) for providing haemostatic compression to a pregnant woman's uterus in a caesarean section procedure during uterine incision, the tool (10) extending in a longitudinal direction from a proximal end (14) to a distal end (12) and having a length defined by the free distance between said proximal and distal ends (14, 12),
the tool (10) comprising
a proximal handle portion (16) extending along a longitudinal handle axis (LA_{H}) towards
a lever portion (20), said lever portion (20) extending along a longitudinal lever axis (LA_{L}) and being proximally adjacently connected to the handle portion (16) and distally adjacently connected to
a foot portion (18), said foot portion (18) extending along a longitudinal foot axis (LA_{F}) and including a proximal foot area (24) as well as two tongues (22) that extend from the proximal foot area (24) in the longitudinal direction and each have a distal free end (26) located at the distal end (12) of the tool (10), the two tongues (22) being positioned at a distance from one another and having an underside (30) forming a common abutment surface (32) for pressing against a pregnant woman's uterus.

2. The surgical tool (10) as claimed in Claim 1, wherein the free distance (29) between the two tongues (22) is at least 1 cm, more preferably at least 1.2 cm, most preferably between 1.5 and 2.5 cm.

3. The surgical tool (10) as claimed in Claim 1 or 2, wherein the two tongues (22) each have a width of at least 0.8 cm, preferably within the range of 1 to 3 cm, said width being preferably essentially constant over the length of the tongues (22).

4. The surgical tool (10) as claimed in any of Claims 1 to 3, wherein the two tongues (22) each have a length of 4 to 8 cm, preferably about 5 to 7 cm, more preferably about 6 cm.

5. The surgical tool (10) as claimed in any of Claims 1 to 4, wherein the abutment surface (32) has a concave shape.

6. The surgical tool (10) as claimed in any of Claims 1 to 5, wherein the abutment surface (32) has a longitudinal cross-sectional shape of an arc, preferably a circular arc.

7. The surgical tool (10) as claimed in Claim 6, wherein a circular segment is enclosed between the arc and its pertaining chord, said segment having a height of at least 4mm, preferably within the range of 5 mm to 10 mm.

8. The surgical tool (10) as claimed any of Claims 1 to 7, wherein - when viewed in longitudinal section - the longitudinal lever axis (LA_{L}) is angularly disposed with relation to the longitudinal handle axis (LA_{H}), the angle at the intersection point being preferably at least 10°, more preferably at angle within the range of 15° to 65°, most preferably within the range of 25° to 45°.

9. The surgical tool (10) as claimed any of Claims 1 to 8, wherein - when viewed in longitudinal section - the longitudinal lever axis (LA_{L}) is angularly disposed with relation to the longitudinal foot axis (LA_{F}), the angle at the intersection point being preferably at least 60°, more preferably at angle within the range of 75° to 120°, most preferably within the range of 85° to 95°.

10. The surgical tool (10) as claimed in any of Claims 1 to 9, wherein the vertical medial plane along the longitudinal lever axis (LA_{L}) is offset from the vertical medial plane along the longitudinal foot axis (LA_{F}), with the two planes intersecting at the connection point of the foot portion (18) and the lever portion (20).

11. The surgical tool (10) as claimed in any of Claims 1 to 10, wherein the lever portion (20) is rigidly connected to, preferably integrally formed with, the handle portion (16) and/or the foot portion (18).

12. The surgical tool (10) as claimed in any of Claims 1 to 11, wherein the two tongues (22) have blunt edges and blunt free ends (26).

13. The surgical tool (10) as claimed in any of Claims 1 to 12, wherein the surgical tool (10) is made of metal or a metal alloy, preferably selected from the group consisting of stainless steel, titanium, tantalum, cobalt and alloys thereof.

14. The surgical tool (10) as claimed in any of Claims 1 to 13, wherein the surgical tool (10) is made from a medical grade plastic material, preferably a medical grade plastic material with a limited contact time on tissue.

15. The surgical tool (10) as claimed in Claim 14, wherein the tongues (22) have a thickness of at least 0.5 cm, preferably at least 0.8 cm, more preferably about 1 cm.
